Europäisches Patentamt

(19) European Patent Office

Office européen des brevets

(11) Publication number : 0 379 428 B1

## (12) EUROPEAN PATENT SPECIFICATION

(45) Date of publication of patent specification :
06.10.93 Bulletin 93/40

(51) Int. Cl.⁵ : **C12N 15/01,** C12N 15/52, C12P 17/18

(21) Application number : 90400129.4

(22) Date of filing : 17.01.90

(54) Improved method for the expression of biotin in bacillus sphaericus.

(30) Priority : 19.01.89 FR 8900597

(43) Date of publication of application :
25.07.90 Bulletin 90/30

(45) Publication of the grant of the patent :
06.10.93 Bulletin 93/40

(84) Designated Contracting States :
CH DE LI

(56) References cited :
AGRIC. BIOL. CHEM., vol. 47, no. 5, 1983,
pages 1011-1016; H. YAMADA et al.: "Biotin
overproduction by biotin analog-resistant mut-
ants of Bacillus sphaericus"
ANNALS OF THE NEW YORK ACADEMY OF
SCIENCES, vol. 447, 1985, pages 335-349; M.A.
EISENBERG: "Regulation of the biotin operon
in E. coli"

(72) Inventor : **Speck, Denis**
**4, rue du Bitzen**
**F-67200 Eckbolsheim (FR)**
Inventor : **Gloeckler, Rémi**
**10, quai Chanoine Winterer**
**F-67000 Strasbourg (FR)**
Inventor : **Lemoine, Yves**
**4, rue des Alisiers**
**F-67100 Strasbourg (FR)**
Inventor : **Ohsawa, Ikuro**
**3-11-11 Nakaochiai Shinjuku-ku**
**Tokyo 161 (JP)**
Inventor : **Kisou, Tugio**
**17-13 Shinohara-nishi koukoku-ku**
**Yokohama 222 (JP)**
Inventor : **Kamogawa, Kouichi**
**2153-42 Kamariya-cho Kanazawa-ku**
**Yokohama 216 (JP)**

(74) Representative : **Warcoin, Jacques et al**
**Cabinet Régimbeau 26, avenue Kléber**
**F-75116 Paris (FR)**

(73) Proprietor : **TRANSGENE S.A.**
**11, rue de Molsheim**
**F-67000 STRASBOURG (FR)**
Proprietor : **NIPPON ZEON CO., LTD.**
**6-1, Marunouchi 2-chome**
**Chiyoda-ku Tokyo 100 (JP)**

EP 0 379 428 B1

## Description

The present invention relates, in particular, to an improved method for the expression of biotin in Bacillus sphaericus.

Biotin or vitamin H is one of the components of the "bios", a complex system of growth factors in yeasts. Within the cell, biotin can be in the free state or strongly bound to proteins; often covalently to the lysine residue of certain enzymes. It is involved in the processes of carbon dioxide transfer, as a prosthetic group of many carboxylases (pyruvate carboxylases, acetyl-CoA carboxylases) or decarboxylases (methylmalonyl-CoA decarboxylase) (Handbook of vitamins, 403 (1984)).

Biotin has been described as a crucial factor for the industrial fermentation of glutamic acid by bacteria (amino acid - nucleic acid, 120 (1968)). There is a relationship between its concentration in the culture medium and the composition of cell membranes, and hence the cell permeability resulting therefrom.

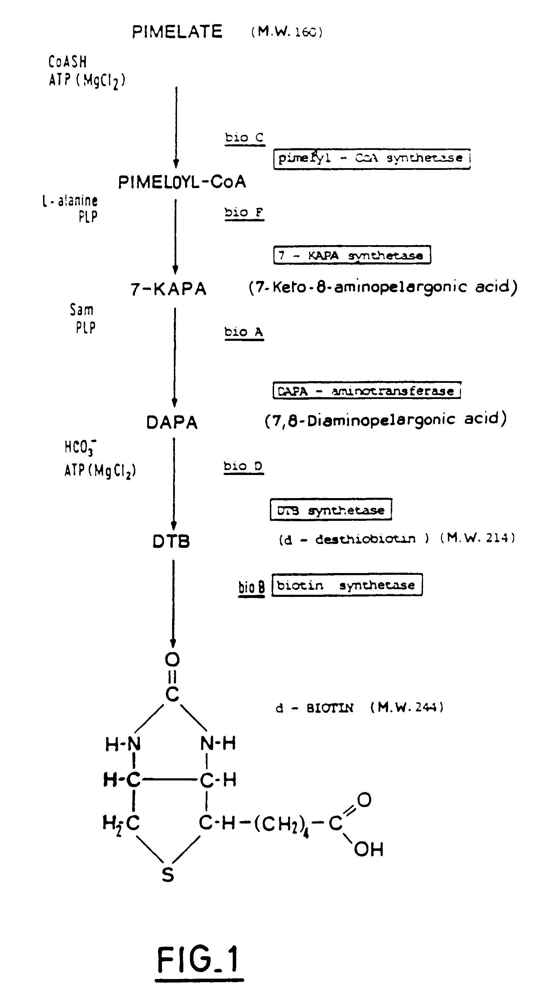

The biotin is synthesized by many microorganisms. Nevertheless, the amounts produced vary greatly according to the species in question. Among bacteria, Bacillus sphaericus is a good potential candidate for the production of biotin from pimelic acid (a precursor of its biosynthesis) during industrial fermentations, since a considerable accumulation of the different intermediates which lead to biotin (hereinafter referred to as "vitamers") is observed in the culture medium (Bull. Inst. Chem. Res. 58 (1980)). Figure 1 shows diagrammatically the pathway of biotin biosynthesis.

Biotin production nevertheless still remains below industrial requirements (Agr. Biol. Chem. 29 No. 10 (1965). This is explained, on the one hand because the biotin produced represses the genes coding for its own synthesis, and on the other hand because the yield of conversion of DTB (desthiobiotin) to biotin is low.

This low bioconversion yield has been improved by amplifying the bioB gene coding for biotin synthetase on a multicopy plasmid of B. sphaericus, as described in Japanese Patent Publication JP 44,889. To abolish the repression exerted by biotin on the expression of the bioB gene, the sequence coding for biotin synthetase has been fused to the $\alpha$-amylase promoter of B. licheniformis. A suitable culture method has been described in US Patent 4,563,426. In this method, pimelic acid is added at the end of the exponential growth phase so as to minimize the repression mechanisms linked to biotin production during the phase of production of the biomass. However, this method only minimizes the repression phenomenon. The present invention relates to the construction of a derepressed strain of B. sphaericus.

The use of derepressed mutants enables the level of expression of all the genes for biotin biosynthesis to be optimized. In effect, for a non-derepressed strain, even without biotin in the culture medium, endogenous biotin synthesis is sufficient to increase its intracellular concentration significantly, and hence to bring about repression of the expression of the biosynthesis genes.

In contrast, in the derepressed mutants according to the invention, all the metabolic fluxes between pimelic acid and biotin can be increased and biotin production enhanced. In addition, the use of derepressed mutants according to the invention enables new fermentation methods to be developed, in which pimelic acid added at the beginning of culturing enables the cells to synthesize biotin during the exponential grown phase, under optimal conditions for the metabolic fluxes.

Many procedures have been described for increasing the quantity of biotin and of vitamers produced by B. sphaericus using mutant strains (Agr. Biol. Chem. 47, 1011 (1983)). Y. Yamada has isolated a collection of mutants resistant to structural analogs of biotin, 5-(2-thienyl)valeric acid and actithiazic acid. For the most important mutants, biotin production is enhanced by a factor of 12, and that of the total vitamers reaches 350 μg/ml (Agr. Biol. Chem. 37, 1079 (1973)). Nevertheless, the exact nature of these mutations still remains unknown, and there are no experimental data in favor of deregulated mutations (Agr. Biol. Chem. 47, 1011 (1983)).

Although alpha-dHB (alpha-dehydrobiotin), another biotin analog, has enabled mutants derepressed for biotin synthesis to be isolated in E. coli Mol. Gen. Genet. 134, 345 1974)), no data describing the production of stable derepressed mutants of B. sphaericus has been published.

To select derepressed mutants, use has often been made of antagonists of metabolites, such as, for example, canavanine for the biosynthesis of arginine (Quant. Biol. 26, 11 (1961)), such as 5-methyltryptophan for that of tryptophan (J. Biol. Chem. 235, 1098 (1960)), such as pyrithiamine for that of thiamine (J. Biochem. 65, No. 3 (1969)) and such as 3-acetylpyrimidine for that of nicotinic acid (Nature, 202, 826 (1964)).

The present invention is based on a new approach for selecting derepressed mutants. It comprises a method for the selection of organisms derepressed in the expression of primary or secondary metabolites, starting with a parent strain of this repressed organism, wherein:

- said parent strain is transformed with a plasmid carrying a marker gene under the control of the DNA sequence involved in the regulation of the expression of said metabolite,
- the strains thus transformed are subjected to a mutagenic treatment, and
- the positive strains expressing said marker in the presence of said metabolite at a concentration totally

EP 0 379 428 B1

repressing the expression of said marker in the transformed parent strain are selected,
- from among these positive strains, the strains hyperproductive of metabolite are selected, optionally after they have been relieved of their plasmids.

In the context of the present invention, the marker is preferably the xylE gene, which has already been described in colorimetric tests (PNAS, 80, 1101 (1983)), and the organism selected is preferably a bacterium, in particular Bacillus sphaericus, and its metabolite biotin, the importance of which has already been described above.

"Derepressed" is understood to denote a strain in which the concentration of primary or secondary metabolite has little or no influence on the production of said metabolite, at all events compared with the repressed parent strain.

The techniques enabling this method to be carried out are known to those versed in the art, and some will be described in greater detail in the examples.

This method, in particular when applied to B. sphaericus for the production of biotin, enables a large number of derepressed strains to be obtained; those which produce the largest amounts of biotin are preferably chosen, although this criterion is not necessarily decisive. It is, in particular, important that the large production of biotin is preserved when the selection plasmid is removed, since it could play the part of a repressor trap and vitiate the results.

The present invention also relates to the strain thereby obtained, as such or as a host strain for plasmid transformations, as well as the strains of B. sphaericus which may be obtained by carrying out this method and which are hyperproductive of biotin.

The present invention also relates to a method for producing biotin by culturing a strain of B. sphaericus producing said biotin in a culture medium, wherein a strain according to the invention which has been transformed with at least one plasmid expressing the bioB gene is cultured and wherein pimelic acid or one of its derivatives is added to the culture medium.

The bioB gene is one of the main genes whose product controls the final stage of biotin biosynthesis, but it is possible to obtain , on the same plasmid or on other auxiliary plasmids, other genes of the biotin biosynthesis chain under the control of regulator sequences, as is described in Patent Publication EP-A-0,266,240. The fermentation conditions are known to those versed in the art, and in the present case, it has been possible to demonstrate an increase in biotin production when pimelic acid or its derivatives is/are added at the beginning of culturing, before or during the exponential growth phase.

Other features and advantages of the invention will become apparent during the detailed description which follows. The examples will be described with reference to the following figures:

Figure 1: Pathway of biotin biosynthesis.
Figure 2: Construction of pTG2414.
Figure 3: Diagram of pTG2414.
Figure 4: Construction of pTG2416.
Figure 5: Diagram of pTG2416.
Figure 6: Construction of pBHB5022.
Figure 7: Construction of pTG498
Figure 8: Expression of bioB on polyacrylamide gel.

Example 1 Construction of plasmids pTG2414 and pTG2416: selection vectors for the isolation of derepressed mutants.

a) Description of plasmid pTG2414

This gram-positive vector contains the fusion of the xylE gene and the regulator region of bioDAYB operon, according to European Patent Publication EP-A-266,240 already cited, the content of which is incorporated herein by reference. The 799-bp NsiI fragment containing the promoter region of the bioDAYB operon was isolated from plasmid pTG1400 and inserted into the PstI site of phage M13TG131 (Gene 26 (1983)).

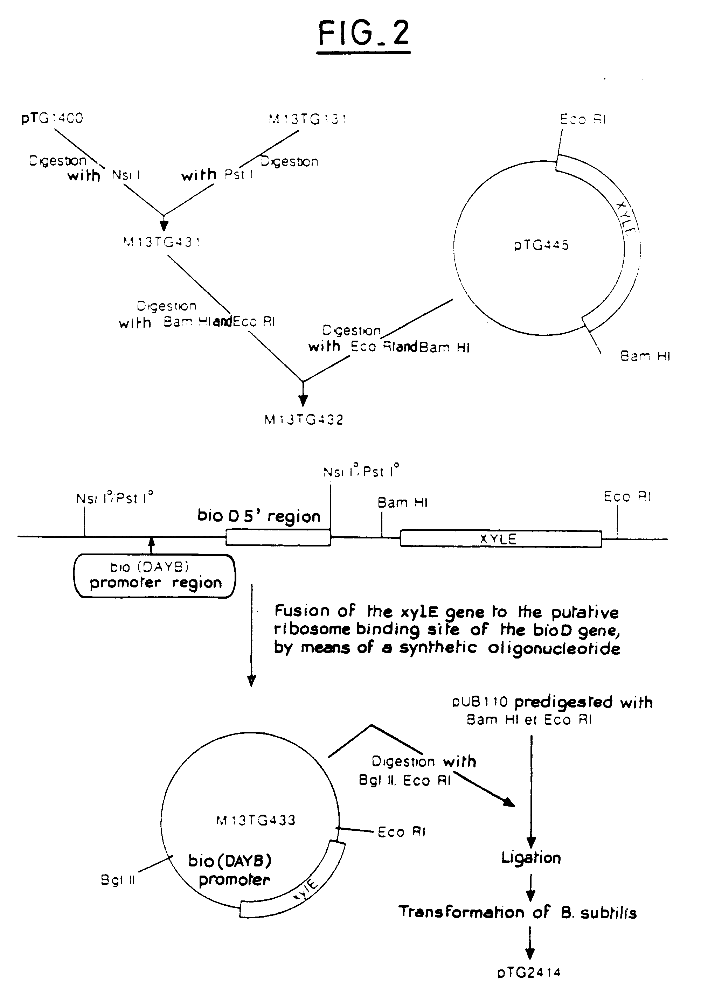

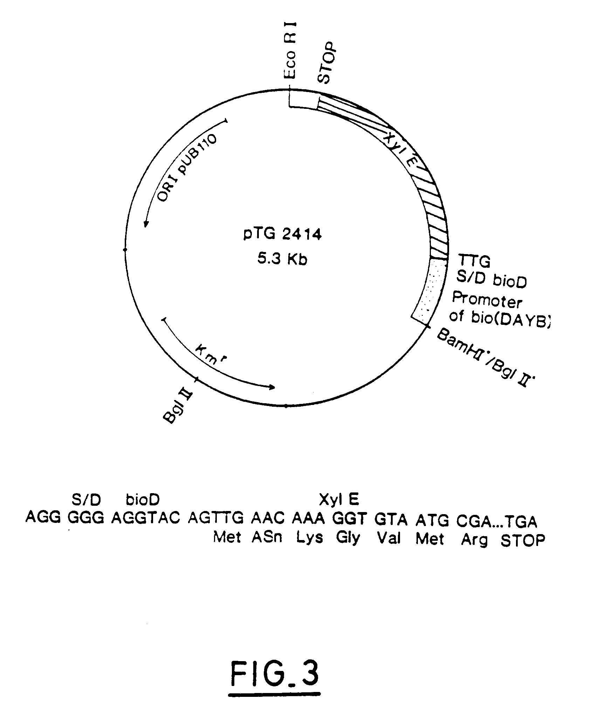

The recombinant phage is referred to as M13TG431. From the vector pTG445 (Genetics and Biotechnology of Bacilli 309 (1984)), the BamHI-EcoRI fragment containing the xylE gene is inserted downstream from the bioDAYB promoter at the BamHI and EcoRI sites of phage M13TG431 (Figure 2). The recombinant phage is referred to as M13TG433. By means of synthetic oligonucleotides, the xylE gene is then fused to the potential ribosome binding site of the bioD gene, as presented in Figure 3.

The BglII-EcoRI fragment containing this fusion was ligated to the replicon pUB110 (J. Bact. 134, 318, 1978), predigested with the enzymes BamHI and EcoRI. The mixture is then incubated in the presence of B. subtilis strain BGSC 1A92 argA2 aroG932 bioB141 sacA321, according to the protocol described by Chang

3

and Cohen (Mol. Gen. Genetic, 168, 1979). The transformants are then selected on DM3 medium supplemented with 100 μg/ml of kanamycin, and transferred by means of a toothpick onto TBAB (Tryptose Blood Agar Base) containing 5 μl/ml of Km. After the dishes are sprayed with 0.5 M catechol solution, the plasmid of the colonies stained yellow is analyzed using the rapid alkaline extraction method (Nucl. Ac. Res., 7, 1513, 1979). The recombinant plasmid containing the DAYB promoter/xylE gene fusion on the replicon pUB110 was referred to as pTG2414.

b) Construction of the vector pTG2416

Plasmid pTG1418 is digested simultaneously with the enzymes HindIII and XmnI. The 1553-bp fragment containing the regulator region of the bioXWF operon is inserted into the HindIII-EcoRV sites of M13TG131. This recombinant phage M13TG432 is then digested with the enzymes EcoRI and SmaI and ligated to the EcoRI-BamHI fragment of pTG445.

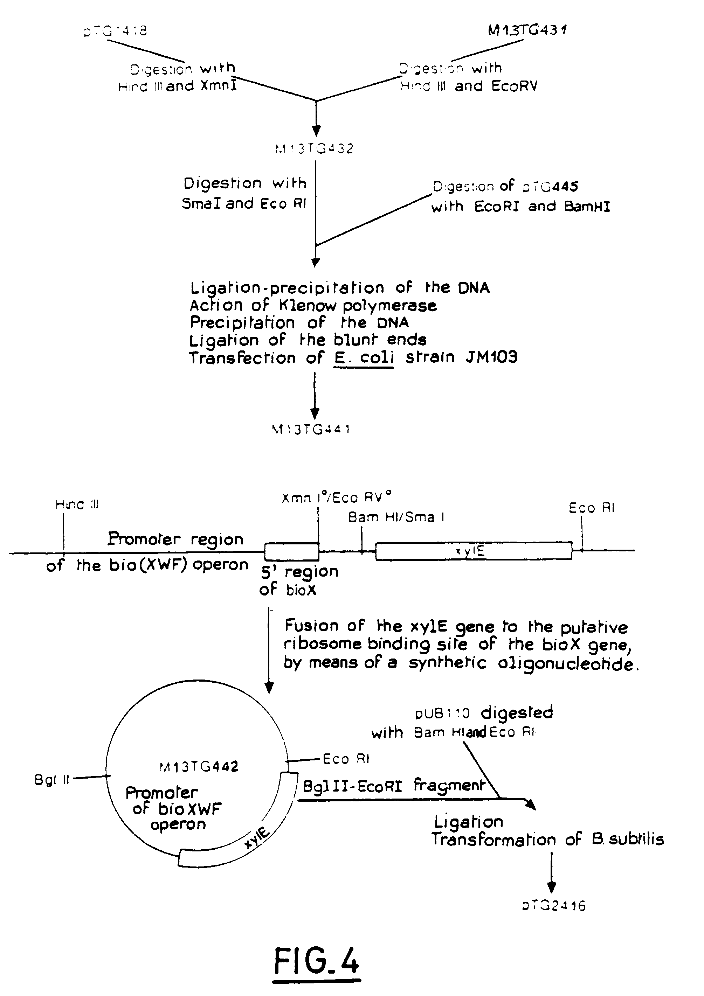

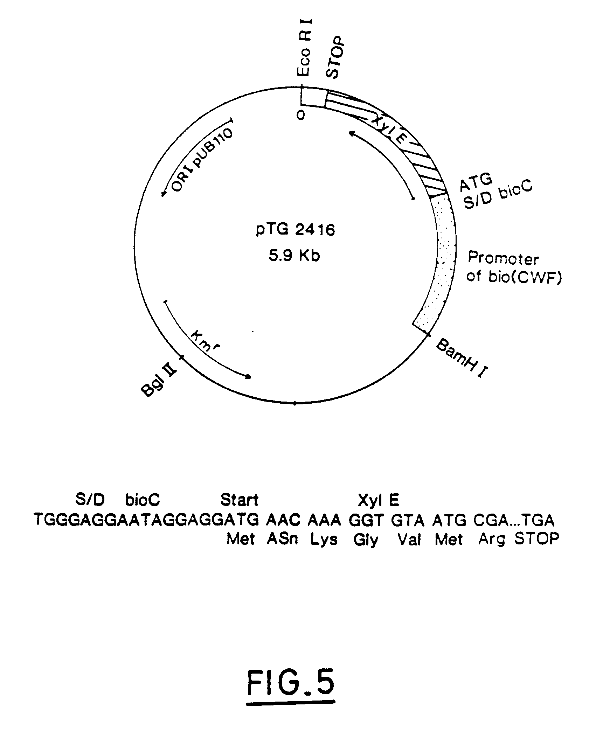

After incubation for 18 h at 15°C, the DNA is precipitated with isopropanol (50:50), and then incubated in the presence of Klenow polymerase (marketed by Boehringer) under the standard conditions described by the manufacturer. The DNA is then precipitated under the same conditions as above and incubated with T4 ligase for 18 hours at 15°C. The recombinant phage is referred to as M13TG441 (Figure 4). Using a synthetic oligonucleotide, the fragment containing the XWF promoter is fused to the xylE structural gene, as shown in Figure 5. The fusion, carried on a BglII-EcoRI fragment of phage M13TG442, was inserted at the EcoRI and BamHI sites of pUB110, giving the vector pTG2416.

Example 2 Transformation of B. sphaericus

B. sphaericus strain IFO 3525 was chosen as a receptor strain for plasmids pTG2414 and pTG2416. The transformation protocol described by Orzech (J. Gen. Microb. 130, 203, 1984)was slightly modified. In effect, lysozyme was replaced by N-acetylmuramidase SG by the production of protoplasts. The transformants were then selected on DM4 regeneration medium containing 150 μg/ml of Km. DM4 corresponds to DM3 (Mol. Gen. Genetic., 168, 111, 1979) slightly modified, and contains 0.66 M succinic acid disodium salt and glycerol instead of succinic acid monosodium salt and glucose, respectively. Finally, the transformants thereby selected were transferred onto GP medium (per liter: glycerol, 20 g; proteose peptone, 30 g; vitamin-free casamino acids, 5 g; $K_2HPO_4$, 1 g; KCl, 0.5g; $MgSO_4.7H_2O$, 0.5 g; $FeSO_4.7H_2O$, 0.01 g; $MnSO_4.4H_2O$, 0.001 g; pH 6.8-7; thiamine-HCl, 20 μg) supplemented with Km (5 μg/ml).

Example 3 Analysis of the phenotype linked to the expression of the xylE gene in terms of the concentration of biotin in the medium

The clones which express the xylE gene become yellow after being sprayed with 0.5 M catechol solution. This staining is attributed to the intracellular conversion of the substrate to a yellow product: 2-hydroxymuconic semialdehyde. The kinetics of appearance of this product depend both on the species and on the level of expression of the xylE gene within the cell. The specific activity of the enzyme encoded by this gene, catechol 2,3-oxygenase ($C_{2,3}O$) may be readily measured, after grinding the cells, by a spectrophotometric assay.

The transformants of strain IFO 3525 are incubated at 37°C on solid GP medium containing different concentrations of biotin, Table 1a. Above a concentration of 50 ng/ml, the expression of the xylE gene is severely repressed. Determination of the specific activity of $C_{2,3}O$ on liquid medium confirms this result, Table 1b.

Table 1: Expression of the xylE gene in the presence of biotin

a. On solid medium: GP + Km 5 $\mu$g/ml

   xylE phenotype: the colonies become yellow after being sprayed with catechol.

| Biotin in ng/ml | 0 | 5 | 10 | 20 | 30 | 40 | 50 | 60 | 80 | 100 |
|---|---|---|---|---|---|---|---|---|---|---|
| Expression of the xylE gene (yellow staining of the cells) | + | + | + | + | + | + | − | − | − | − |

b. On liquid medium: GP + Km 5 $\mu$g/ml

   Measurement of the activity of the xylE gene product ($C_{2,3}O$) of destroyed cells cultured in the presence of an increasing concentration of biotin.

| Biotin in ng/ml | 0 | 1 | 2 | 5 | 10 | 50 | 100 |
|---|---|---|---|---|---|---|---|
| Specific activity of $C_{2,3}O$ in mU/mg | | 91 | 41 | 30 | 32 | 24 | 25 | 1 |

Example 4 Isolation of a derepressed strain

To isolate a derepressed strain, it is necessary to obtain a collection of mutants capable of expressing the xylE gene constitutively, starting with transformants of IFO 3525 containing either pTG2414 or pTG2416.

Thus, transformants containing pTG2414 were cultured in liquid GP medium for 4 to 5 hours, and the collected cells were suspended in 0.01 M phosphate buffer. Then were then treated with 100 μg/ml of nitrosoguanidine for 5 to 10 minutes at 37°C.

After this mutagenic treatment, the cells were washed in phosphate buffer, collected and plated out on GP medium containing 10 μg/ml of biotin, and incubated for 18 h at 37°C. The dishes were then sprayed with 0.5 M catechol solution; the cells becoming yellow represented potential deregulated mutants.

The colonies stained yellow were isolated, and each colony was cultured for 2 days at 37°C in GP medium containing 1 mg/ml of pimelic acid. The amount of biotin accumulated for each strain is given in Table 2.

## Table 2: Production of biotin by the derepressed strains

| Strain | Biotin $\mu$g/ml |
|---|---|
| TK 105-1 | 0.34 |
| TK 105-3 | 0.50 |
| TK 212-1 | 0.58 |
| TK 212-3 | 0.73 |
| TK 212-4 | 0.87 |
| TK 226-1 | 0.50 |
| TK 226-10 | 0.46 |
| TK 502-2 | 1.00 |
| TK 502-6 | 0.82 |
| TK 502-8 | 0.90 |
| IFO 3525-pTG 2414 | max 0.1 |
| IFO 3525 | 0.11 |

Among the mutant strains which produce more biotin than the wild-type strain IFO 3525, TK 502-2 was subjected to the protocol described below in order to relieve it of its plasmid. After incubation in LB medium at 37°C for 18 hours, 1/100th of the volume of the culture was inoculated into another LB medium, freshly prepared and supplemented with 0 to 10 $\mu$g/ml of novobiocin, and incubated for 18 hours.

The cultures which exhibit slightly retarded growth compared with those without novobiocin were plated out on solid GP medium and incubated for 18 hours at 37°C. The colonies which did not stain yellow after treatment with 0.5 M catechol were then selected, and the DNA extracted from each strain analyzed by electrophoresis. The strains which lost pTG2414 were tested for their capacity to accumulate biotin, and the strain TK 502-2-C5 was adopted.

Example 5 Analysis of the deregulated strain

We limited our study to the strain TK 502-2-C5 on account of its capacity to produce biotin after being cultured in the presence of precursor from the beginning of culturing and in the absence of plasmid pTG2414 (which could act as a repressor trap). This strain, in which pTG2414 and pTG2416 have been reintroduced, still expresses the xylE gene constitutively in the presence of biotin, confirmed by the yellowing in the presence of catechol of colonies transformed on GP medium. This experiment clearly establishes that a modification of the wild-type strain has taken place, that the mutation is localized at chromosomal level and that the deregulation affects both bioDAYB and bioXWF operons. To quantify the level of derepression of such a strain, we measured the specific activity of $C_{2,3}O$ from cells cultured in the presence or absence of biotin on GP medium. Two conclusions are to be drawn from Table 3:
- The specific activity of $C_{2,3}O$ is only slightly influenced by the presence of biotin in TK 502-2-C5 compared with the wild-type strain.
- The specific activity of $C_{2,3}O$ is amplified even in the absence of biotin.

6

Table 3:  Specific  activity  of  $C_{2,3}O$  of  B. sphaericus  strain TK 502-2-C5

| Strain | Plasmid | Specific activity of $C_{2,3}O$ mU/mg | |
|---|---|---|---|
| | | in GP medium | in GP medium + biotin (10 $\mu$g/ml) |
| IFO 3525 | pTG2414 | 85 | 2 |
| TK 502-2-C5 | pTG2414 | 480 | 340 |
| IFO 3525 | pTG2416 | 60 | 1 |
| TK 502-2-C5 | pTG2416 | 140 | 45 |

These results suggest that the specific activity of each biotin enzyme has increased in this mutant.

Example 6 Amplification of the bioB gene in the deregulated strain of B. sphaericus

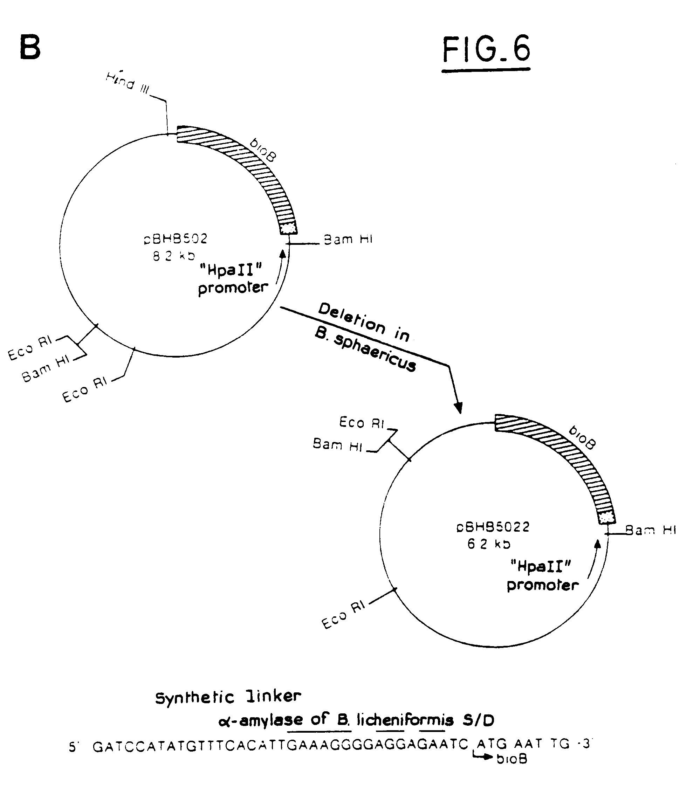

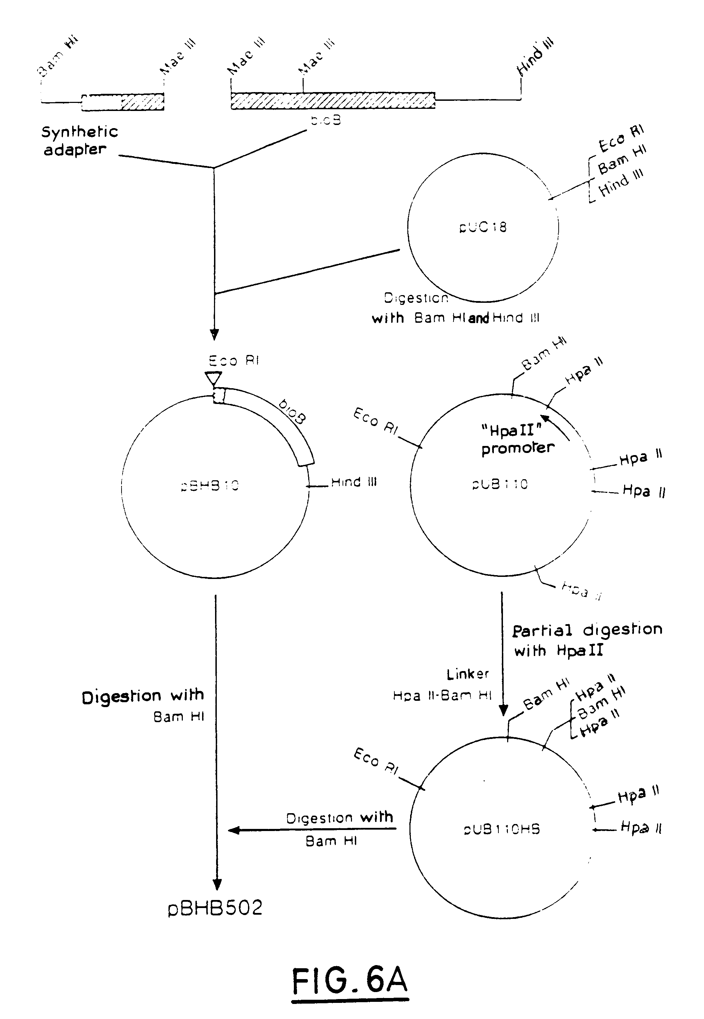

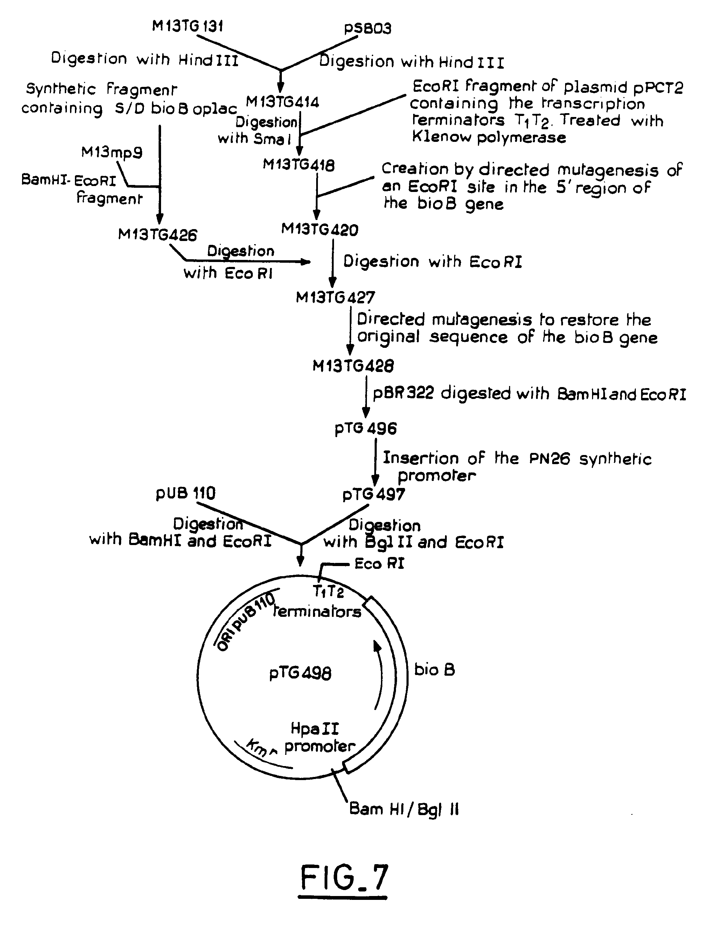

The expression vectors of bioB, pBHB5022 and pTG498, were constructed as described in Figures 6 and 7. TK 502-2-C5 was transformed with these vectors and the amount of biotin produced by the transformants is presented in Table 4.
Two main conclusions may be drawn:
- The amplification of the bioB gene is a good factor for increasing the yield of biotin in a deregulated strain.
- The conversion of DTB to biotin is considerable during the exponential growth phase, and it is easier to add pimelic acid from the beginning of culturing.

Table 4:  Production of biotin by the derepressed strain  TK 502-2-C5 of B. sphaericus

| Strain | Biotin in $\mu$g/ml | |
|---|---|---|
| | Pimelic acid added at t = 0 | Pimelic acid added at t = 24 h |
| IFO 3525 | 0.01 | 0.50 |
| IFO 3525/pBHB5022 | nd | 13.30 |
| TK 502-2-C5 | 1.50 | 0.60 |
| TK 502-2-C5/pTG498 | 10-30 | 4.50 |
| TK 502-2-C5/pBHB5022 | 20-45.5 | 9.10 |

After inoculation, the cultures were grown for 1 day in GP medium at 30°C, and then at 37°C for 2 days. Biotin production in the supernatant is assessed by the microbiological assay of L. plantarum ATCC8014. In the case of the derepressed strains, the cultures were maintained at 37°C from the beginning of the experiments. Pimelic acid 0.5 mg/ml, nd = not determined.

Analysis of the expression of bioB on polyacrylamide gel

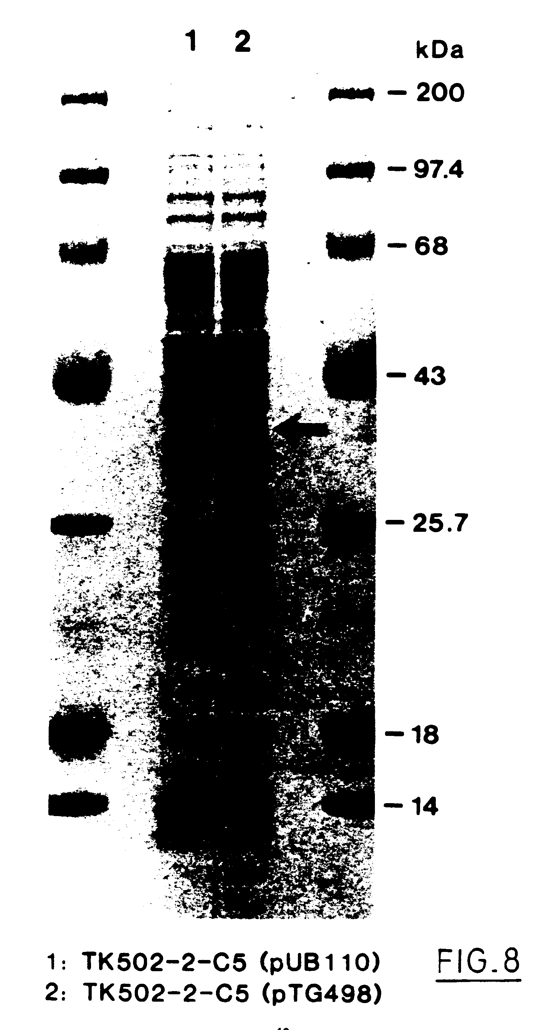

B. sphaericus TK 502-2-C5 (pTG498) cells are incubated for 18 h at 37°C in GP medium containing 10 µg/ml of kanamycin. 100 µl of culture were centrifuged in an Eppendorf tube, and the pellet after washing was treated according to the protocol described by Peschke (J. Mol. Biol. 186, 1985). 5 µl were tested on 13% polyacrylamide gel. After migration for 5 h at 20 mA, the gel is fixed in a solution containing 7.5% acetic acid/5% methanol/0.25% Coomassie blue, and visualized in a 7.5% acetic acid/5% methanol solution. Figure 8 shows the appearance of a new band with an apparent molecular mass of 37 kD obtained from the extract containing the amplified bioB gene. These results demonstrate the good correlation between the increase in biotin production and the expression of biotin synthetase.

B. sphaericus strain TK 502-2-C5 was deposited on November 8, 1988, at the Collection Nationale de Culture de Microorganismes (National Collection of Microorganism Cultures) of the Pasteur Institute, 28 Rue du Docteur Roux, 75724 Paris Cedex, under No. I-808.

## Claims

1. A method for the selection of organisms derepressed in the expression of primary or secondary metabolites, starting with a parent strain of this normally repressed organism, wherein:
   - said parent strain is transformed with a plasmid carrying a marker gene under the control of the DNA sequence involved in the regulation of the expression of said metabolite,
   - the strains thus transformed are subjected to a mutagenic treatment, and
   - the positive strains expressing said marker in the presence of said metabolite at a concentration totally repressing the expression of said marker in the transformed parent strain are selected,
   - from among these positive strains, the strains hyperproductive of metabolite are selected, optionally after they have been relieved of their plasmids.

2. The method as claimed in claim 1, wherein the metabolite is biotin and the marker gene is the xylE gene, the strain being Bacillus sphaericus.

3. A strain of depressed organisms which are obtained by carrying out the method as claimed in one of claims 1 and 2.

4. A strain of B. sphaericus derepressed in the expression of biotin, which can be obtained by carrying out the method as claimed in claim 2.

5. A method for producing biotin by culturing a strain of B. sphaericus producing said biotin in a culture medium, wherein a strain as claimed in claim 4 which has been transformed with at least one plasmid expressing the bioB gene is cultured and wherein pimelic acid or one of its derivatives is added to the culture medium.

6. The method for producing biotin as claimed in claim 5, wherein said strain has been transformed with a plasmid expressing other genes of the biotin biosynthesis chain.

7. The method as claimed in one of claims 5 and 6, wherein pimelic acid or its derivatives is/are added at the beginning of culturing, before or during the exponential growth phase.

8. The process as claim in one of claims 5 to 7, wherein the strain is B. sphaericus strain CNCM No. I-808.

## Patentansprüche

1. Verfahren zur Selektion in der Expression primärer oder sekundärer Metabolite dereprimierter Organismen, beginnend mit dem elterlichen Stamm dieses normalerweise reprimierten Organismus, wobei:
   - der elterliche Stamm unter der Steuerung einer DNA-Sequenz, die bei der Regulation der Expression des Metaboliten beteiligt ist, mit einem ein Marker-Gen tragenden Plasmid transformiert wird,
   - die so transformierten Stämme einer Mutagen-Behandlung unterworfen werden, und
   - die positiven Stämme, die den Marker in der Anwesenheit des Metaboliten bei einer Konzentration, welche die Expression des Markers in dem transformierten elterlichen Stamm vollständig reprimiert, exprimieren, selektiert werden,
- von diesen positiven Stämmen die metabolit-hyperproduktiven Stämme selektiert werden, gegebenenfalls nachdem sie von ihren Plasmiden befreit worden sind.

2. Verfahren nach Anspruch 1, wobei der Metabolit Biotin und das Marker-Gen das xylE-Gen ist, wobei der Stamm Bacillus sphaericus ist.

3. Stamm dereprimierter Organismen, welche durch Durchführung des Verfahrens gemäß einem der Ansprüche 1 oder 2 erhalten sind.

4. Stamm von B. sphaericus, welches in der Expression von Biotin dereprimiert ist, der durch Durchführung des Verfahrens nach Anspruch 2 erhalten werden kann.

5. Verfahren zur Herstellung von Biotin durch Kultivation eines Stammes von B. sphaericus, welcher das Biotin produziert, in einem Kulturmedium, wobei ein Stamm nach Anspruch 4, welcher mit wenigstens einem Plasmid, welches das bioB-Gen exprimiert, transformiert ist, kultiviert wird, und wobei Pimelinsäure oder eines ihrer Derivate dem Kulturmedium hinzugegeben wird.

6. Verfahren zur Herstellung von Biotin nach Anspruch 5, wobei der Stamm mit einem Plasmid, welches andere Gene der Biotin-Biosynthesekette exprimiert, transformiert worden ist.

7. Verfahren nach einem der Ansprüche 5 oder 6, wobei Pimelinsäure oder ihre Derivate zu Beginn der Kultivation, vor oder während der exponentiellen Wachstumsphase hinzugegeben wird bzw. werden.

8. Verfahren nach einem der Ansprüche 5 bis 7, wobei der Stamm der B. sphaericus Stamm CNCM Nr. I-808 ist.

## Revendications

1. Procédé de sélection d'organismes déréprimés dans l'expression de métabolites primaires ou secondaires, à partir d'une souche de base de cet organisme normalement réprimé, caractérisé en ce que :
   - on transforme ladite souche de base avec un plasmide portant un gène marqueur sous le contrôle de la séquence d'ADN impliquée dans la régulation de l'expression dudit métabolite,
   - on soumet les souches ainsi transformées à un traitement mutagène, et
   - on sélectionne les souches positives exprimant ledit marqueur en présence dudit métabolite à une concentration réprimant en totalité l'expression dudit marqueur chez la souche de base transformée,
   - parmi ces souches positives, on sélectionne les souches hyperproductrices de métabolite, éventuellement après avoir été débarrassées de leurs plasmides.

2. Procédé selon la revendication 1, caractérisé en ce que le métabolite est la biotine et le gène marqueur est le gène xylE, la souche étant Bacillus sphaericus.

3. Souche d'organismes déréprimés obtenus par la mise en oeuvre du procédé selon l'une des revendications 1 et 2.

4. Souche de B. sphaericus déréprimée dans l'expression de la biotine pouvant être obtenue par la mise en oeuvre du procédé selon la revendication 2.

5. Procédé de production de biotine par culture d'une souche de B. sphaericus produisant ladite biotine dans un milieu de culture, caractérisé en ce qu'on cultive une souche selon la revendication 4 qui a été transformée par au moins un plasmide exprimant le gène bioB et en ce qu'on ajoute l'acide pimélique ou l'un de ses dérivés au milieu de culture.

6. Procédé de production de biotine selon la revendication 5, caractérisé en ce que ladite souche a été transformée par un plasmide exprimant d'autres gènes de la chaine de biosynthèse de la biotine.

7. Procédé selon l'une des revendications 5 et 6, caractérisé en ce que l'acide pimélique, ou ses dérivés, est ajouté au début de la culture, avant ou pendant la phase exponentielle de croissance.

8. Procédé selon l'une des revendications 5 à 7, caractérisé en ce que la souche est la souche de B. sphaericus n° I-808.

PIMELATE   (M.W. 160)

CoASH
ATP (MgCl₂)

bio C

pimelyl - CoA synthetase

PIMELOYL-CoA

L - alanine
PLP

bio F

7 - KAPA synthetase

7-KAPA   (7-Keto-8-aminopelargonic acid)

Sam
PLP

bio A

DAPA - aminotransferase

DAPA   (7,8-Diaminopelargonic acid)

HCO₃⁻
ATP (MgCl₂)

bio D

DTB synthetase

DTB   (d - desthiobiotin) (M.W. 214)

bio B   biotin synthetase

d - BIOTIN   (M.W. 244)

# FIG.1

# FIG_2

S/D    bioD                           Xyl E
AGG GGG AGGTAC AGTTG AAC AAA GGT GTA ATG CGA...TGA
                                Met ASn Lys Gly Val Met Arg STOP

# FIG. 3

FIG.4

14

S/D   bioC         Start              Xyl E
TGGGAGGAATAGGAGGATG AAC AAA GGT GTA ATG CGA...TGA
                    Met ASn Lys Gly  Val Met Arg STOP

# FIG.5

B                                                    FIG.6

Synthetic linker
α-amylase of B. licheniformis S/D

5' GATCCATATGTTTCACATTGAAAGGGGAGGAGAATC ATG AAT TG ·3'
                                          → bioB

16

FIG. 6A

M13TG131                                    pSB03

Digestion with Hind III        Digestion with Hind III

Synthetic fragment                    EcoRI fragment of plasmid pPCT2
containing S/D bio B oplac  M13TG414   containing the transcription
                           Digestion   terminators T₁T₂. Treated with
M13mp9                     with Sma I   Klenow polymerase

BamHI-EcoRI                M13TG418    Creation by directed mutagenesis of
fragment                               an EcoRI site in the 5' region of
                                       the bioB gene

M13TG426              M13TG420

        Digestion          Digestion with EcoRI
        with Eco RI

            M13TG427

            Directed mutagenesis to restore the
            original sequence of the bioB gene

            M13TG428

            pBR322 digested with BamHI and EcoRI

            pTG 496

            Insertion of the PN26 synthetic
                          promoter

pUB 110              pTG 497

Digestion            Digestion
with BamHI and EcoRI with Bgl II and EcoRI

                    Eco RI

                T₁T₂
            terminators

        pTG 498            bio B

            Hpa II
            promoter

        Bam HI / Bgl II

## FIG_7

1: TK502-2-C5 (pUB110)
2: TK502-2-C5 (pTG498)

FIG.8